# EUROPEAN PATENT APPLICATION

(11) **EP 1 230 911 A1**
(43) Date of publication of application: **14.08.2002**
(21) Application number: 02075586.4
(22) Date of filing: 12.02.2002
(51) Int. Cl.: A61K 7/48, A61K 7/06, A61K 7/025, A61K 35/78, A61K 35/64

(54) **cosmetic or pharmaceutical preparation**

(30) Priority: 12.02.2001 NL 1017333
(71) Applicant: Van Gent Natural Products, 3233 ZG Oostvoorne (NL)
(72) Inventor: Van Gent, Louis, 3233 BC Oostvoorne (NL)
(74) Representative: Jorritsma, Ruurd

(57) **Abstract**

The invention relates to a cosmetic or pharmaceutical preparation that contains the following five imperative ingredients: (1) honey (extract), (2) aloe vera extract, (3) vitamin E, (4) Calendula officialis and (5) methylsulphonylmethane. For this combination of ingredients it has been found that this displays a synergistic action with regard to the recovery of irritated or damaged skin and produces a surprising improvement, in terms of speed and quality, in the normal skin structure. In addition to the abovementioned five imperative ingredients, the preparations, which can be processed to give a water-in-oil or oil-in-water emulsion, ointment, cream, soap, shampoo, bath or shower gel or lip balm, can also contain other ingredients, such as lanolin, coconut fatty acid or sunflower oil.

## Description

The invention relates to a cosmetic or pharmaceutical preparation.

Cosmetic preparations usually have a restoring, softening and conditioning action on the skin. For example, in the case of a dry to very dry skin the associated irritations are reduced and the aim is for complete skin recovery.

Furthermore, preparations can be used in the role of pharmaceutical preparations in the repair of damage to the skin. Such damage to the skin is referred to as a wound. This can be a cut, a stab wound, bruising (caused by a blow or otherwise) or a laceration.

A wide variety of preparations are known for effecting or promoting the recovery of damaged skin. In this context these preparations can be based on diverse ingredients.

In this context one group of medicinal substances for promoting recovery of the skin after treatment is based on natural ingredients. More particularly, four natural substances are described below, i.e.:
- honey or honey extract; this substance is generally known to possess a skin-restoring or wound-healing action;
- aloe vera extract; this material is also known to possess a skin-restoring or wound-healing action;
- vitamin E; this substance is also known to have a skin-restoring or wound-healing action;
- Calendula officialis extract; research has revealed that this substance displays skin-restoring or wound-healing activity; and
- methylsulphonylmethane, which likewise has a skin-restoring activity.

With regard to, for example, the natural substance honey it is pointed out for the sake of completeness that
a) this contains an appreciable concentration of the enzyme glucose oxidase; on dilution of the honey, this enzyme, which originates from the salivary gland of bees, converts glucose to gluconic acid and hydrogen peroxide, which latter substance has a bactericidal action;
b) this contains an adequate concentration of glucose and fructose; because of these two substances honey attracts water and is therefore able to draw pus, including bacteria and toxins remaining therein, from the wound; and
c) it has a low water content of approx. 16% to 17%; as a result of the low water vapour pressure (a/w value) of honey, honey is able to kill bacteria. This is because the majority of bacteria and moulds are no longer able to absorb water at an a/w value of less than 0.90, so that they die off in the course of time. In honey the a/w value varies between 0.45 and 0.70.

Aloe vera extracts are described as being able to suppress atypical or juvenile eczemas. Furthermore, the use thereof as a cosmetic preparation for the skin and as a pharmaceutical preparation for the treatment of burns is known.

Vitamin E can be applied directly to the skin, especially in the case of sunburn or other types of burns. Vitamin E is also used for leg ulcers and other skin ailments.

Calendula officialis, an extract derived from marigolds in water, is used for healing wounds and cracks in the skin.

Furthermore, as far as methylsulphonylmethane is concerned it is known that the use of this in the case of skin disorders such as acne and the like gives a favourable result.

Surprisingly it has now been found that a combination of the ingredients honey, aloe vera extract, vitamin E, Calendula officialis and methylsulphonylmethane displays a synergistic action with regard to the recovery of irritated or damaged skin and brings about a surprising improvement, in terms of speed and quality, of the normal skin structure, associated with a general improvement in the condition of the skin. The invention therefore relates to a preparation which is characterised in that the composition comprises
(1) honey or honey extract,
(2) aloe vera extract,
(3) vitamin E,
(4) Calendula officialis, and
(5) methylsulphonylmethane.

In addition to the abovementioned five imperative ingredients, the preparation according to the invention can contain other ingredients such as, for example, lanolin, and in particular coconut fatty acid and sunflower oil. This latter substance is able, by reason of the unsaturated fatty acids content, to capture the free oxygen radicals occurring in a wound, which radicals would otherwise attack the other healthy cell membranes. Depending on the quantity and type of the ingredients in the preparations according to the invention, these preparations can be processed to give a water-in-oil emulsion, oil-in-water emulsion, ointment, cream, shampoo, soap, bath or shower gel as well as a lip balm.

More particularly, the preparation according to the invention comprises
1) 3 - 80 % (m/m) honey
2) 1 - 80 % (m/m) aloe vera extract
3) 1 - 20 % (m/m) vitamin E
4) 1 - 40 % (m/m) Calendula officialis, and
5) 1 - 25 % (m/m) methylsulphonylmethane, as well as, optionally,
6) 1 - 50 % (m/m) coconut fatty acid and/or
7) 1 - 50 % (m/m) sunflower oil.

Advantageously, the preparation is in the form of an ointment containing 1 - 20 % (m/m) sunflower oil.

### Example I

A preparation according to the invention can be prepared as follows.

100 g honey is mixed together with 10 g aloe vera extract for 5 minutes in a stirrer/mixer at a temperature of 30 °C. The ingredients vitamin E (10 g), Calendula officialis extract (10 g) and methylsulphonylmethane (10 g) are then added to the mixture and the whole is mixed for 10 min. The preparation therefore consists of:
71.4 % (m/m) honey
7.15 parts by weight aloe vera extract
7.15 parts by weight vitamin E
7.15 parts by weight Calendula officialis extract and
7.15 parts by weight methylsulphonylmethane.

## Claims

1. Cosmetic or pharmaceutical preparation comprising (1) honey or honey extract, (2) aloe vera extract, (3) vitamin E, (4) Calendula officialis and (5) methylsulphonylmethane.

2. Preparation according to Claim 1, **characterised in that** the preparation also contains coconut fatty acid.

3. Preparation according to Claim 1 or 2, **characterised in that** the preparation contains sunflower oil.

4. Preparation according to one or more of Claims 1 - 3, **characterised in that** the preparation comprises
1) 3 - 80 % (m/m) honey
2) 1 - 80 % (m/m) aloe vera extract
3) 1 - 20 % (m/m) vitamin E
4) 1 - 40 % (m/m) Calendula officialis, and
5) 1 - 25 % (m/m) methylsulphonylmethane, as well as, optionally,
6) 1 - 50 % (m/m) coconut fatty acid and/or
7) 1 - 50 % (m/m) sunflower oil.

5. Preparation according to one or more of Claims 1 - 4, **characterised in that** the preparation is processed to give a water-in-oil emulsion, oil-in-water emulsion, ointment, cream, shampoo, bath or shower gel or lip balm.

6. Preparation according to one or more of Claims 1 - 5, **characterised in that** the preparation is in the form of an ointment which also contains 1 - 20 % (m/m) sunflower oil.
